(19) Europäisches Patentamt / European Patent Office / Office européen des brevets

(11) **EP 3 758 786 B1**

(12) **EUROPEAN PATENT SPECIFICATION**

(45) Date of publication and mention
of the grant of the patent:
**23.04.2025 Bulletin 2025/17**

(21) Application number: **19760283.2**

(22) Date of filing: **04.03.2019**

(51) International Patent Classification (IPC):
*A61M 35/00* (2006.01)    *A61B 90/80* (2016.01)

(52) Cooperative Patent Classification (CPC):
**A61M 35/006; A61L 2/0088; A61L 2/26;**
A61L 2202/14; A61L 2202/15

(86) International application number:
**PCT/US2019/020483**

(87) International publication number:
**WO 2019/169376 (06.09.2019 Gazette 2019/36)**

(54) **MEDICAL DEVICE WITH INTEGRATED SENSING TECHNOLOGY FOR REDUCING HUMAN FACTOR ERROR DURING PRE-SURGICAL SKIN ANTISEPSIS**

MEDIZINISCHES GERÄT MIT INTEGRIERTER SENSORTECHNOLOGIE ZUR REDUZIERUNG EINES HUMANFAKTORFEHLERS WÄHREND DER PRÄOPERATIVEN HAUTANTISEPTIK

DISPOSITIF MÉDICAL À TECHNOLOGIE DE DÉTECTION INTÉGRÉE POUR RÉDUIRE UNE ERREUR DE FACTEUR HUMAIN PENDANT UNE ANTISEPSIE CUTANÉE PRÉ-CHIRURGICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.03.2018 US 201815910318**

(43) Date of publication of application:
**06.01.2021 Bulletin 2021/01**

(73) Proprietor: **CAREFUSION 2200, INC**
**San Diego, CA 92130 (US)**

(72) Inventors:
• **YARGER, Michael**
**San Diego, California 92130 (US)**
• **RADFORD, Robert**
**San Diego, California 92130 (US)**

(74) Representative: **Banchetti, Marina et al**
**Barzanò & Zanardo S.p.A.**
**Via Piemonte 26**
**00187 Roma (IT)**

(56) References cited:
WO-A1-2017/109519    US-A- 4 483 626
US-A1- 2007 276 312    US-A1- 2012 316 381
US-A1- 2014 309 686    US-A1- 2015 134 357
US-A1- 2016 030 233    US-A1- 2017 318 964
US-A1- 2018 161 560

## Description

**The current application claims priority to** United States Application No. 15/910,318, entitled "Housing with Integrated Sensing Technology for Reducing Human Factor Error During Pre-Surgical Skin Antisepsis," filed on March 2, 2018**, which is a continuation-in-part of** United States Application No. 15/377,092, entitled "Antiseptic Applicator," filed on December 13, 2016**.**

### Field

The present disclosure relates generally to antiseptic applicators.

## BACKGROUND

Clinician practices related to cleaning skin of a patient prior to a procedure, such as an intravenous catheter insertion or surgery, may be inconsistent and may deviate from protocol and recommended guidelines. In further detail, clinicians may not clean an insertion or surgical site for a sufficient amount of time to remove unwanted bacteria prior to the insertion or the surgery. Failure to clean the insertion or surgical site for the sufficient amount of time may result in an increased likelihood of infection for the patient. For example, a patient's chances of developing a catheter-related bloodstream infection ("CRBSI") and/or surgical site infection ("SSI") may increase. CRBSIs and SSIs are responsible for increased health care costs. Accordingly, there is a need in the art for devices and methods that facilitate cleaning of the insertion site for the sufficient amount of time.

The international patent publn. No. WO2017/109519 discloses an apparatus for topical application of material for cosmetic or medical purposes, said apparatus comprising measurement apparatus configured to measure a property of the human or animal skin, actuating apparatus configured to change a property of the skin and an application apparatus configured to apply material for cosmetic or medical purposes to the skin.

## SUMMARY

The following presents a simplified summary of one or more features described herein in order to provide a basic understanding of such features. This summary is not an extensive overview of all contemplated features, and is intended to neither identify key or critical elements of all features nor delineate the scope of any or all implementations. Its sole purpose is to present some concepts of one or more features in a simplified form as a prelude to the more detailed description that is presented later.

The present invention provides a medical device for sterilizing a body of a patient, as recited in the appended set of claims.

An antiseptic applicator according to the invention includes an applicator body configured to store antiseptic solution, an antiseptic dispenser in communication with the applicator body, and a capacitance sensing integrated circuit having one or more electrically coupled capacitors, wherein the capacitance sensing integrated circuit is configured to determine a capacitance between the one or more capacitors and a human body.

A method of sterilizing the skin of a patient with an antiseptic applicator includes dispensing an antiseptic solution from the antiseptic applicator into a dispenser of the antiseptic applicator, measuring a capacitance between the dispenser and a human body, determining a capacitance between the dispenser and the human body, triggering a timer to record an amount of time when the capacitance is approximately greater than or equal to a threshold capacitance, and terminating the timer when the capacitance falls below the threshold capacitance.

A medical device for sterilizing the body of a patient according to the invention includes an applicator body that stores antiseptic solution, an antiseptic dispenser that dispenses the antiseptic solution, a capacitor abutting a surface of the antiseptic dispenser, and measurement features for determining a capacitance between the capacitor and the body of the patient.

The foregoing has outlined rather broadly the features and technical advantages of examples according to the disclosure in order that the detailed description that follows may be better understood. Additional features and advantages will be described hereinafter. The conception and specific examples disclosed may be readily utilized as a basis for modifying or designing other structures for carrying out the same purposes of the present disclosure. Such equivalent constructions do not depart from the scope of the appended claims. Characteristics of the concepts disclosed herein, both their organization and method of operation, together with associated advantages will be better understood from the following description when considered in connection with the accompanying figures. Each of the figures is provided for the purpose of illustration and description only, and not as a definition of the limits of the claims.

## BRIEF DESCRIPTION OF THE DRAWINGS

Figure 1 is an upper perspective view of an antiseptic applicator according to the invention;
Figure 2 is a flow chart of an example method of using the antiseptic applicator to clean the skin of the patient;
Figure 3 is an exploded view of another embodiment of antiseptic applicator according to the invention;
Figure 4 is a block diagram of circuit components in the antiseptic applicator of Figure 3;
Figure 5 is a schematic circuit diagram of the antiseptic applicator of Figure 3;
Figure 6 is an example capacitance measurement of the capacitors in the antiseptic applicator of Figure 3;
Figure 7 is an example computer that may be used with the antiseptic applicators of the present disclosure;
Figure 8 shows an example communication system for use in accordance with an aspect of the present invention; and
Figure 9 is a flow chart of example methods of using the antiseptic applicator of the invention.

## DETAILED DESCRIPTION

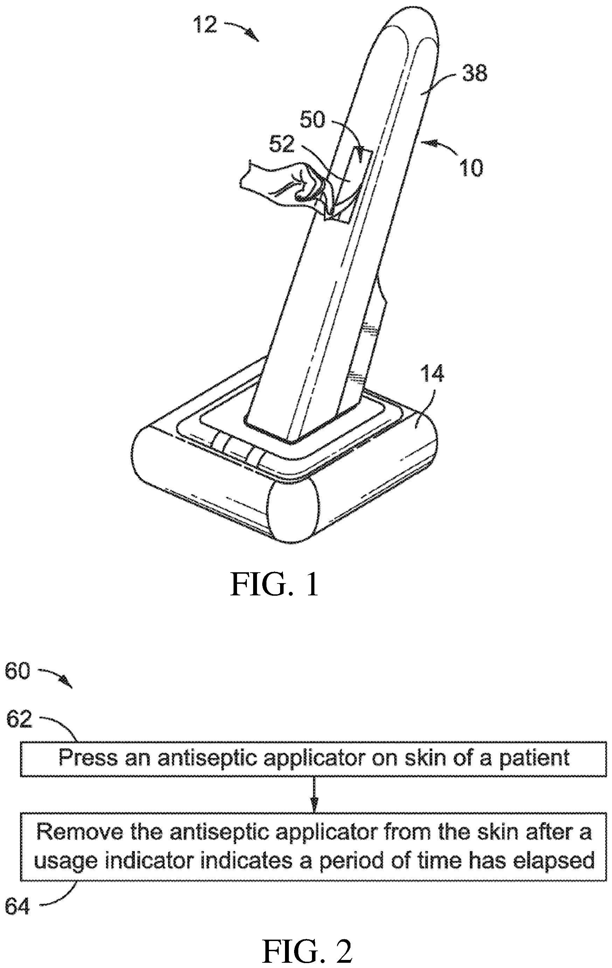

With reference to Figure 1, an antiseptic solution is disposed within a handle 10 of an antiseptic applicator 12. The handle 10 stores the antiseptic solution. The antiseptic applicator 12 may be used to clean or sterilize skin of a patient, for example. The antiseptic applicator 12 may be used to clean the skin prior to a procedure such as, for example, intravenous catheter insertion. The antiseptic applicator 12 may include a sponge 14, which may be configured to absorb the antiseptic solution when the antiseptic solution is released from the handle 10. The antiseptic applicator 12 includes a usage indicator, which is configured to indicate a period of time that the antiseptic applicator 12, and thus, the antiseptic solution, has been applied to the skin of the patient.

Various types of usage indicators may be used. Referring to Figure 1, the antiseptic applicator 12 may include a color-changing element 50 as the usage indicator antiseptic applicator 12. In some embodiments, the color-changing element 50 may include a peelable color-changing element 52, which may include an outer liner that may be removed or partially removed or peeled. In response to the outer liner 54 being removed or partially removed, one or more chemicals of the peelable color-changing element 52 may change color upon exposure to air for a particular amount of time. In some embodiments, the peelable color-changing element 52 may be configured such that the change in color occurs after the predetermined period of time. In some embodiments, the clinician may peel the outer liner 54 of the peelable color-changing element 52 immediately prior to or immediately after pressing the antiseptic applicator 12 on the skin of the patient and may continuously press the antiseptic applicator 12 on the skin until the change in color occurs. The change in color may indicate to the clinician that the clinician has pressed the antiseptic applicator 12 to the skin of the patient for the predetermined period of time. For example, the predetermined period of time may be between thirty and sixty seconds. The peelable color-changing element 52 may be various shapes and sizes. In some embodiments, the peelable color-changing element 52 may be configured as a strip, as illustrated in Figure 1.

In some embodiments, the color-changing element 50 may include a reservoir of one or more chemicals. The reservoir may be configured such that when the clinician presses on the reservoir, the chemicals may mix, which may cause a color of the usage indicator to change after the predetermined period of time. The clinician may press the reservoir immediately prior to or immediately after pressing the antiseptic applicator 12 on the skin of the patient and may be able to determine when the antiseptic applicator 12, and thus, the antiseptic solution 40 (not illustrated in Figure 1), has been pressed to the skin for a sufficient period of time to prevent infection.

In some embodiments, the antiseptic applicator 12 may include a timer, which may be digital or analog, which may be activated by the clinician immediately prior to or immediately after pressing the antiseptic applicator 12 to the skin of the patient. The timer may count the predetermined period of time, such as, for example, thirty seconds or sixty seconds. The timer may include an alarm, which may sound when the predetermined period of time has elapsed. In some embodiments, the timer may be disposed in a position on the antiseptic applicator 12 that may be easily visible to the clinician, such as on an upper portion of the handle 10.

Figure 2 illustrates a block diagram of an example method 60 of using an antiseptic applicator to clean the skin of the patient, according to some embodiments. The antiseptic applicator may include the antiseptic applicator 12. The method 60 may begin at block 62. At block 62, the antiseptic applicator may be pressed on the skin of the patient. In some embodiments, pressing the antiseptic applicator on the skin of the patient may also include scrubbing the antiseptic applicator on the skin of the patient. Block 62 may be followed by block 64.

At block 64, the antiseptic applicator may be removed from the skin after the usage indicator indicates a period of time has elapsed, for example, the predetermined period of time. The usage indicator may include one or more LEDs, each of which may indicate the period of time by one or more of the following: changing color, turning on, and turning off. The usage indicator may include a color-changing element, such as, for example, a peelable color-changing element, which may indicate the period of time by changing color.

Although illustrated as discrete blocks, various blocks may be divided into additional blocks, combined into fewer blocks, or eliminated, depending on the desired implementation. The method 60 may include additional blocks. For example, the method 60 may include peeling a color-changing element prior to or shortly after pressing the antiseptic applicator on the skin.

In addition to the previously described embodiments of the antiseptic applicators 12, 50, each of the antiseptic applicators 12, 50 may be modified in any suitable manner that allows it to fulfill its intended purpose. Further, the antiseptic applicators 12, 50 may be used in any suitable manner.

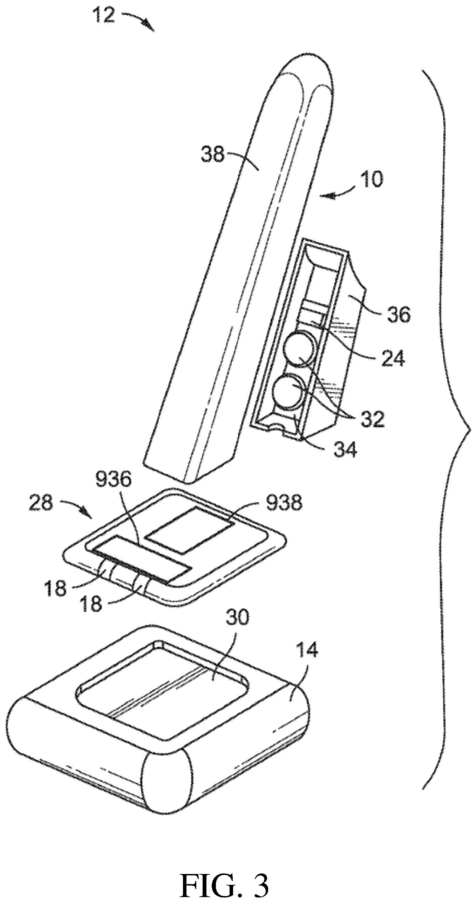

Referring now to Figure 3, the antiseptic applicator 12 relies on capacitive sensors to detect proper contact. Reference numbers similar to those in previous figures indicate similar features. In certain implementations, the usage indicator may include one or more lights, such as LEDs 18. In these and other embodiments, the antiseptic applicator further includes one or more capacitors 936, which may be disposed in the proximity of the sponge 14. The one or more capacitors 936 may be configured for use in detecting any contact and/or close proximity between the antiseptic applicator 12 and the skin of the patient. The antiseptic applicator 12 also includes a timer 24, which may be electrically coupled to the usage indicator and/or the one or more capacitors 936, which may be disposed in close proximity to the sponge 950. When the antiseptic applicator 12 contacts the skin of the patient (e.g., via the sponge 950), the effect of the close proximity of the human body to the circuitry containing the one or more capacitors 936 may trigger the timer 24 or other device, such as an alert or indicator light. The one or more capacitors 936 may be disposed on top of the coupler element 28, beneath the coupler element 28, on top of the sponge 14, beneath the sponge, or embedded within the sponge. Other configurations are possible.

In some embodiments, the timer 24 is responsive to output from the circuit containing the one or more capacitors 936. The output from the circuit containing the one or more capacitors 936 may result from the antiseptic applicator 12 contacting or being placed in close proximity to the skin of the patient. For example, the timer may begin to display or internally track elapsed time in response to an output from the circuit containing the one or more capacitors 936. As another example, the timer 24 may start displaying or tracking the elapsed time in response to the output from the circuit containing the one or more capacitors 936 meeting or exceeding (or alternatively falling below) a predetermined threshold value. The timer 24 may stop tracking or displaying elapsed time when the output from the one or more capacitors 936 meets or falls below another predetermined threshold value (or alternatively exceeds the threshold value). In yet another example, the timer 24 may retain tracked elapsed time when the timer 24 stops tracking the elapsed time, such that when the output again meets or exceeds the threshold value, the time 24 may start from the tracked elapsed time value instead of from zero. Alternatively, in an example, the timer 24 may retain tracked elapsed time until it is reset through any number of triggering features or events, or methods, such as clinician powering off the antiseptic applicator 12, automatic resetting of the timer 24 when a predetermined period of time has passed, or clinician manually resetting the timer 24.

In example embodiments, when the elapsed time equals a predetermined period of time, one or more LEDs 18 may be turned on or change color to indicate to the clinician that a desired minimum cleaning duration has been achieved.

In some implementations, one of the LEDs 18 may be turned on in response to contacts or close proximity between the antiseptic applicator 12 and the skin of the patient detected by the one or more capacitors 936. Next, another one of the LEDs 18 may be turned on in response to the elapsed time being equal to or greater than the predetermined period of time. The predetermined period of time may correspond to a length of time to create a contact between the sponge 14 having antiseptic thereon and the skin of the patient according to improved or best practices for infection prevention. For example, the predetermined period of time may be 5 seconds, 10 seconds, 20 seconds, 30 seconds, 45 seconds, 60 seconds, 90 seconds, and 120 seconds. Other durations are possible.

As shown in Figure 3, in example embodiments, an aperture 938 may extend through the coupler element 28. The antiseptic solution may be configured to flow from the handle 10 to the sponge 14 through the aperture 938 when the antiseptic solution is released from the handle 910. The antiseptic solution may be released from the handle 910 via any number of mechanisms and/or methods. For example, the bottom of the handle 910 may include an opening or conduit through which the antiseptic solution may be configured to flow at a predetermined rate. Alternatively, the handle 910 may include a switch that, when pressed, releases the antiseptic solution via an opening or conduit (e.g., via opening of a valve therein). Other configurations are possible.

In alternative embodiments, the one or more capacitors 936 may surround the aperture 938. For example, the coupler element 930 may be disposed within a depression 952 in an upper surface of the sponge 950. For example, the coupler element 930 may be configured to fit snuggly within the depression 952 of the sponge 950. The one or more LEDs 932 may be disposed on the coupler element 930. For example, the one or more LEDs 932 may be disposed on an edge of the coupler element 930.

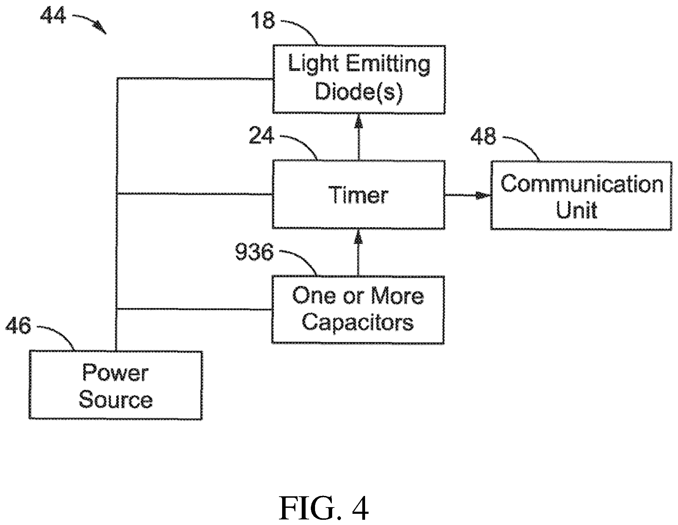

Alternatively, the one or more LEDs 932 may be disposed in various positions on the antiseptic applicator 900 that allows the clinician to view the one or more LEDs 932. Referring now to Figure 4, the circuit components 44 of the antiseptic applicator 12 may include a power source 46, the one or more LEDs 18, the timer 24, the circuitry containing one or more capacitors 936 for use in proximity or contact sensing, and/or a communication circuit 48. Reference numbers similar to those in previous figures indicate similar features.

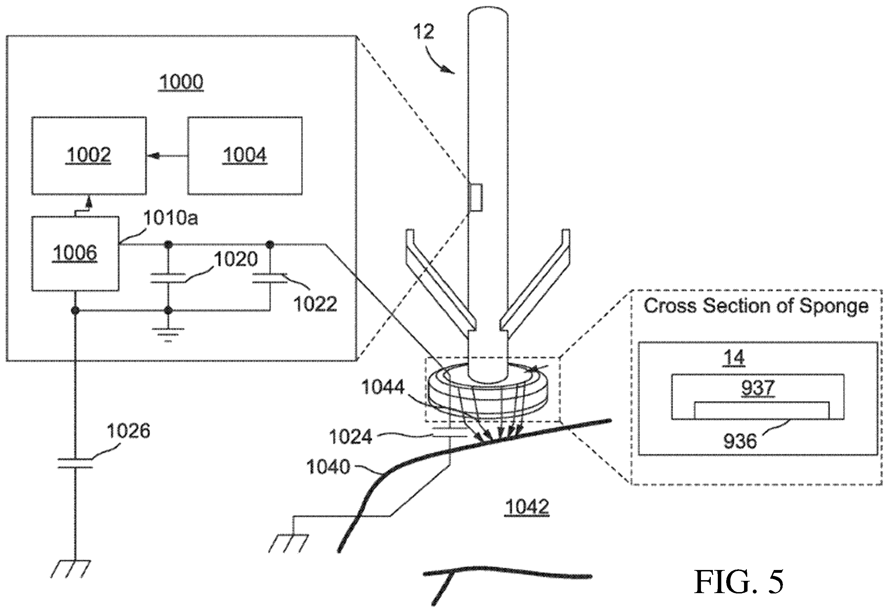

Referring now to Figure 5, in some embodiments, the antiseptic applicator 12 may include an integrated circuit 1000 having a microprocessor 1002, an accelerometer 1004, and/or elements of a capacitive sensing integrated circuit (CSIC) 1006. The microprocessor 1002 may be or include a semiconductor microprocessor, a field programmable gate array, a programmable logic device, and/or any suitable processor, for example. The accelerometer 1004 may include a damping mass used to measure an acceleration based on the displacement of the damping mass, for example. The accelerometer 1004 may be implemented, for example, using piezoelectric, piezoresistive, capacitive, or micro-electromechanical components. Other components or combination of components are possible. Examples of suitable accelerometer 1004 are described in BMA 200 Digital, Triaxial Acceleration Sensor Data Sheet, the content of which is incorporated by reference in its entirety.

In some implementations, the one or more capacitors 936 may be partially encased in a shield 937, which may be disposed on top of or beneath, or embedded within the sponge 14. The shield 937 may protect the one or more capacitors 936 from corrosion, oxidation, or other kinds of damages. Further, the shield 937 may reduce electromagnetic noise detected by the one or more capacitors 936. The shield may be a coating, metallic casing, ceramic shell, or other suitable configurations.

In some implementations, the CSIC elements 1006 may be electrically coupled to the one or more capacitors 936 of the antiseptic applicator 12 to measure the capacitance of an applicator capacitor 1024 formed between the one or more capacitors 936 and the body of a patient 1042. As the one or more capacitors 936 are moved closer to a skin 1040 of the patient 1042, for example, the body of the patient 1042 may begin to distort the electric field lines 1044 of the circuitry containing the one or more capacitors 936. This distortion may change the capacitance of the one or more capacitors 936. For example, the capacitance of the circuitry containing the capacitor(s) 936 may increase when the one or more capacitors 936 move closer to the patient's skin 1040. By reading the capacitance of the one or more capacitors 936, the CSIC 1006 may determine the proximity of the antiseptic applicator 12 with respect to the skin 1040.

In example embodiments, the one or more capacitors 936 may be configured as a single capacitor. Alternatively, the one or more capacitors 936 may be configured as parallel plates capacitors and/or parallel fingers electrodes, for example. Other similarly operating configurations may also be used.

In some embodiments, the CSIC 1006 may be configured to detect the capacitance change on the one or more capacitors 936. During the capacitance measurement, the CSIC 1006 may measure the capacitance values of the applicator capacitor 1024, a parasitic capacitor 1020 and an electrode capacitor 1022. For example, a capacitance measurement taken by the CSIC 1006 at a sensing node 1010a may include a sum of the parasitic capacitor 1020, the electrode capacitor 1022, and the applicator capacitor 1024, or any combination thereof. The parasitic capacitor 1020 and the electrode capacitor 1022 may be or include intrinsic capacitors that originate from the hardware configuration of the integrated circuit 1000. The parasitic capacitor 1020 and the electrode capacitor 1022 may be measured or estimated. Examples of suitable capacitance sensing circuits are described in FDC 1004: Basics of Capacitive Sensing and Applications, the content of which is incorporated by reference in its entirety.

During normal operation, for example, the CSIC 1006 may apply a sensing voltage ($V_{Sense}$) at the sensing node 1010a. The sensing voltage may be a direct current (DC) voltage, an alternating current (AC) voltage, or a combination of AC and DC voltage. The CSIC 1006 may measure (e.g., via sensor or coupling) a sensing charge ($Q_{Total}$) at the sensing node 1010a. Since the applicator capacitor 1024, the parasitic capacitor 1020, and the electrode capacitor 1022 are in parallel configuration, the CSIC 1006 receives at the sensing node 1010a an equivalent capacitance value ($C_{Total}$) approximately equaling to the sum of the capacitance values of the parasitic, electrode, and applicator capacitors 1020, 1022, 1024. Further, since

$$C_{Total} \approx C_{Parasitic} + C_{Electrode} + C_{Applicator},$$

and

$$Q = C \times V \text{ generally,}$$

the equivalent capacitance equation may be rewritten as follows:

$$\frac{Q_{Total}}{V_{Sense}} \approx C_{Parasitic} + C_{Electrode} + \frac{Q_{Applicator}}{V_{Sense}},$$

assuming the impact of a ground capacitor 1026 is negligible. Since $V_{Sense}$, $Q_{Total}$, $C_{Parasitic}$, and $C_{Electrode}$ are known, the capacitance value of the applicator capacitor 1024 may be approximated by the following equation:

$$\frac{Q_{Total}}{V_{Sense}} - (C_{Parasitic} + C_{Electrode}) \approx C_{Applicator}.$$

In example implementations, the CSIC 1006 may extrapolate the distance between the one or more capacitors 936 and the skin 1040 of the patient 1042 based on the capacitance value of the applicator capacitor 1024. The applicator capacitance may be approximated by:

$$C_{Applicator} \approx A\frac{\varepsilon}{d},$$

where A is the cross-sectional area of the one or more capacitors 936, $\varepsilon$ is the effective dielectric constant between the one or more capacitors 936 and the skin 1040, and d is the distance between the one or more capacitors 936 and the skin 1040. Using the capacitance equation, the CSIC 1006 may extract the distance, d, between the one or more capacitors 936 and the skin 1040 and/or the CSIC 1006 may simply identify when the distance is less than a minimum distance indicative of contact with the skin 1040. For example, as the antiseptic applicator 900, and therefore the one or more capacitors 936, moves closer to the skin 1040, the applicator capacitance may increase due to the decrease in distance d. Similarly, the applicator capacitance may decrease as the antiseptic applicator 12 moves away from the skin 1040. When the distance d is less than a threshold distance, the CSIS 1006 may determine that the antiseptic applicator 900 is in contact with the patient's skin 1040.

Alternatively, the CSIC 1006 may compare the applicator capacitance to a threshold capacitance indicative of contact with the skin 1040. For example, as the antiseptic applicator 900 moves closer to the skin 1040, the applicator capacitance may increase. When the applicator capacitance meets or exceeds the threshold capacitance, the CSIC 1006 may determine that the antiseptic applicator 900 is in contact with the patient's skin 1040.

In some embodiments, the CSIC 1006 may transmit the measured applicator capacitance to the microprocessor 1002. In this example, the microprocessor 1002 may identify the distance between the one or more capacitors 936 and the skin 1040 of the patient 1042 or otherwise determine that the distance is below a threshold distance that corresponds to contact between the antiseptic applicator 12 and the patient's skin 1040.

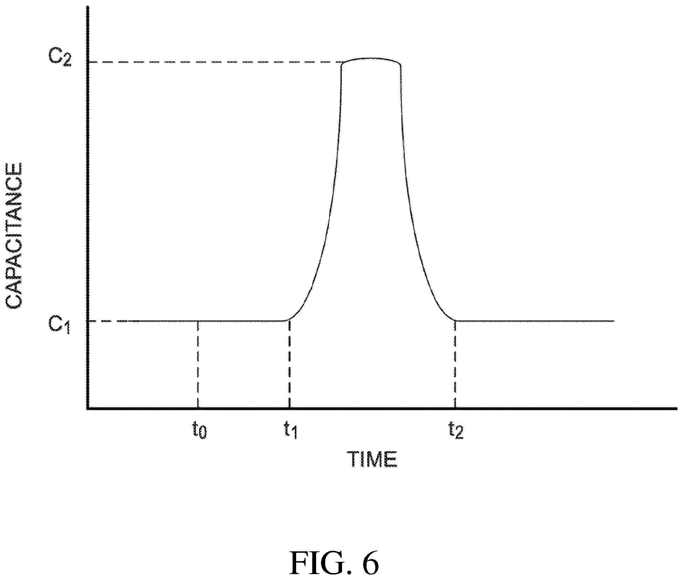

Referring now to Figure 6, for example, at time $t_0$ the clinician may have the antiseptic applicator 900 located away from the patient 1042, and the CSIC 1006 may measure an applicator capacitance value of $C_1$. At $t_1$, with reference to Figure 5, the clinician may move the one or more capacitors 936 closer to the skin 1040 of the patient 1042 by, for example, placing the sponge 14 of the antiseptic applicator 900 against the skin 1040 of the patient to begin the sterilization process. Consequently, CSIC 1006 of Figure 5 may measure an applicator capacitance value of $C_2$ at some point between $t_1$ and $t_2$. At $t_2$, with reference to Figure 5, the clinician may end the sterilization by moving the antiseptic applicator 900 away from the skin 1040 of the patient, and the CSIC 1006 may measure the decrease in applicator capacitance back to $C_1$.

Returning to Figure 5, in certain implementations, the CSIC 1006 may be electrically coupled to the accelerometer 1004 to detect the motion of the antiseptic applicator 900. For example, the accelerometer 1004 may detect a "back-and-forth" scrubbing motion of the antiseptic applicator 12. In another example, the accelerometer 1004 may detect a circular scrubbing motion of the antiseptic applicator 12. Other possible motions, such as shaking, may also be detected by the accelerometer 1004.

In some implementations, the microprocessor 1002 may collect data from the accelerometer 1004 and/or the CSIC 1006 to determine the sufficiency of the sterilization process. For example, the microprocessor 1002 may rely on the accelerometer data to determine and/or signal if the clinician engaged in back-and-forth scrubbing during the sterilization process. In another example, the microprocessor 1002 may utilize data from the CSIC 1006 to determine and/or signal if the clinician cleaned the skin 1040 of the patient for a sufficient period of time. In yet another example, the microprocessor 1002 may analyze data from the accelerometer 1004 and the CSIC 1006 to determine and/or signal if the clinician performed circular scrubbing for a certain duration while the sponge 14 of the antiseptic applicator 900 remained in contact with the skin 1040. In another example, the microprocessor 1002 may determine and/or signal, using data from the accelerometer 1004 and the CSIC 1006, if the clinician performed both back-and-forth and circular scrubbing. Other analyses and/or signals are also possible.

In example embodiments, the integrated circuit 1000 may be coupled to the circuit components 970 and/or receive electrical power from the power source 972. The integrated circuit 1000 may be electrically connected to the communication circuit 974, for example. During normal operation, the integrated circuit 1000 may send data from the microprocessor 1002, the accelerometer 1004, and/or the CSIC 1006 to the communication circuit 974 to be transmitted to an external device, such as a smart telephone, tablet, computer or other suitable devices via Bluetooth, Near Field Communication (NFC), Radio Frequency Identification (RFID), Wi-Fi, or any other communication technology. The data may include whether or not the clinician applied the antiseptic applicator 900 to the skin 1040 of the patient 1042 for the predetermined period of time, an amount of time the clinician applied the antiseptic applicator 900 to the skin 1040 of the patient 1042, whether or not the clinician properly scrubbed the skin 1040 of the patient 1042 during the sterilization process, an failure relating to the integrated circuit 1000, the microprocessor 1002, the accelerometer 1004, or the CSIC 1006, or any combination thereof. Other data relevant to the operation of the antiseptic applicators may also be transmitted via the communication unit 947. The transmitted data may be monitored for safety and/or compliance purposes. For example, a supervisor may rely on the data to monitor whether the clinician properly cleaned the skin of a patient.

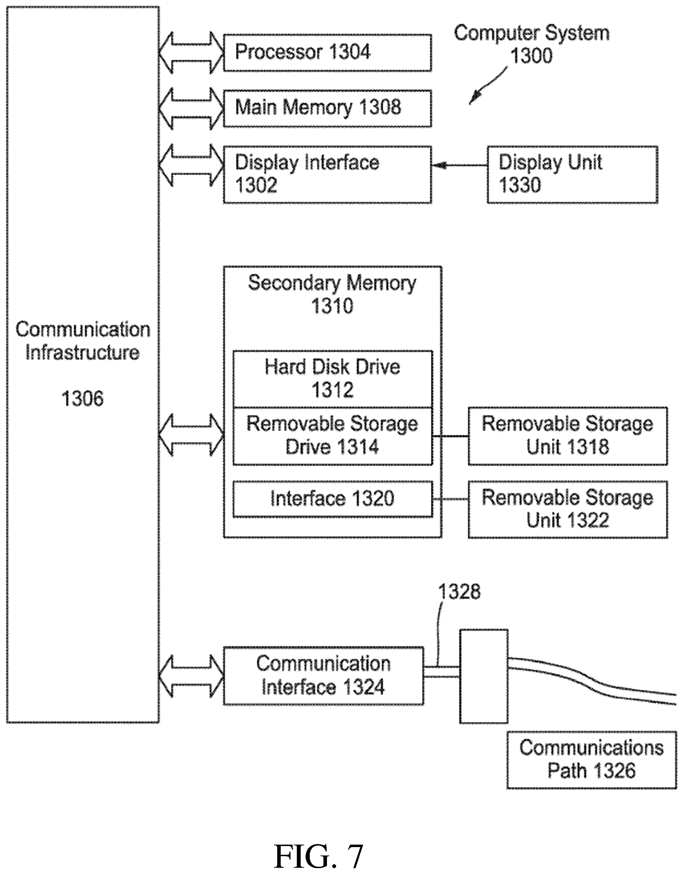

Aspects of the present disclosure may be implemented using hardware, software, or a combination thereof and may be implemented in one or more computer systems or other processing systems. In an aspect of the present disclosure, features are directed toward one or more computer systems capable of carrying out the functionality described herein. In some embodiments the integrated circuit 1000 may be implemented as a part of a computer system, or may include various aspects a computer system, such as the example computer system 1300 shown in Figure 7. Computer system 1300 includes one or more processors, such as processor 1304. The processor 1304 is coupled to a communication infrastructure 1306 (e.g., a communications bus, cross-over bar, or network). Various software aspects are described in terms of this example computer system. After reading this description, it will become apparent to a person skilled in the relevant art(s) how to implement aspects hereof using other computer systems and/or architectures.

Computer system 1300 may include a display interface 1302 that forwards graphics, text, and other data from the communication infrastructure 1306 (or from a frame buffer not shown) for display on a display unit 1330. Computer system 1300 may include a main memory 1308, preferably random access memory (RAM), and may also include a secondary memory 1310. The secondary memory 1310 may include, for example, a hard disk drive 1312 and/or a removable storage drive 1314, representing a floppy disk drive, a magnetic tape drive, an optical disk drive, etc. The removable storage drive 1314 may read from and/or write to a removable storage unit 1318 in a well-known manner. Removable storage unit 1318, represents a floppy disk, magnetic tape, optical disk, etc., which may be read by and written to removable storage drive 1314. As will be appreciated, the removable storage unit 1318 may include a computer usable storage medium having stored therein computer software and/or data.

Alternative aspects of the present invention may include secondary memory 1310 and may include other similar devices for allowing computer programs or other instructions to be loaded into computer system 1300. Such devices may include, for example, a removable storage unit 1322 and an interface 1320. Examples of such may include a program cartridge and cartridge interface (such as that found in video game devices), a removable memory chip (such as an erasable programmable read only memory (EPROM), or programmable read only memory (PROM)) and associated socket, and other removable storage units 1322 and interfaces 1320, which allow software and data to be transferred from the removable storage unit 1322 to computer system 1300.

Computer system 1300 may also include a communications interface 1324. Communications interface 1324 may allow software and data to be transferred among computer system 1300 and external devices. Examples of communications interface 1324 may include a modem, a network interface (such as an Ethernet card), a communications port, a Personal Computer Memory Card International Association (PCMCIA) slot and card, etc. Software and data transferred via communications interface 1324 may be in the form of signals 1328, which may be electronic, electromagnetic, optical or other signals capable of being received by communications interface 1324. These signals 1328 may be provided to communications interface 1324 via a communications path (e.g., channel) 1326. This path 1326 may carry signals 1328 and may be implemented using wire or cable, fiber optics, a telephone line, a cellular link, a radio frequency (RF) link, Wi-Fi link, Wi-Fi direct link, NFC, and/or other communications channels. As used herein, the terms "computer program medium" and "computer usable medium" refer generally to media such as a removable storage drive 1380, a hard disk installed in hard disk drive 1370, and/or signals 1328. These computer program products may provide software to the computer system 1300. Aspects of the present invention are directed to such computer program products.

Computer programs (also referred to as computer control logic) may be stored in main memory 1308 and/or secondary memory 1310. Computer programs may also be received via communications interface 1324. Such computer programs, when executed, may enable the computer system 1300 to perform the features in accordance with aspects of the present invention, as discussed herein. In particular, the computer programs, when executed, may enable the processor 1310 to perform the features in accordance with aspects of the present invention. Accordingly, such computer programs may represent controllers of the computer system 1300.

Where aspects of the present invention may be implemented using software, the software may be stored in a computer program product and loaded into computer system 1300 using removable storage drive 1314, hard drive 1312, or communications interface 1320. The control logic (software), when executed by the processor 1304, may cause the processor 1304 to perform the functions described herein. In another aspect of the present invention, the system may be implemented primarily in hardware using, for example, hardware components, such as application specific integrated circuits (ASICs) and/or microcontrollers. Implementation of the hardware state machine so as to perform the functions described herein will be apparent to persons skilled in the relevant art(s).

In yet another variation, aspects of the present invention may be implemented using a combination of both hardware and software.

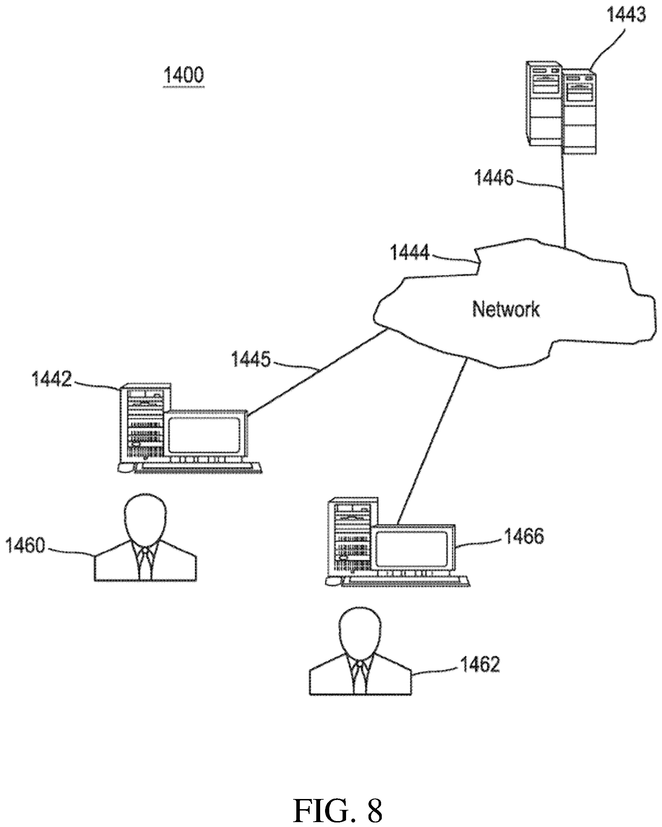

Figure 8 shows a communication system 1400 usable in accordance with aspects of the present invention. The communication system 1500 includes one or more accessors 1460, 1462 (also referred to interchangeably herein as one or more "users") and one or more terminals 1442, 1466. In one aspect, data for use in accordance with the present invention is, for example, input and/or accessed by accessors 1460, 1462 via terminals 1442, 1466, such as personal computers (PCs), minicomputers, mainframe computers, microcomputers, telephonic devices, or wireless devices, such as personal digital assistants ("PDAs"), smart phones, or other hand-held wireless devices coupled to a server 1443, such as a PC, minicomputer, mainframe computer, microcomputer, or other device having a processor and a repository for data and/or connection to a repository for data, via, for example, a network 1444, such as the Internet or an intranet, and couplings 1445, 1446, 14164. The couplings 1445, 1446, 1464 include, for example, wired, wireless, or fiberoptic links. In another variation, the method and system in accordance with aspects of the present invention operate in a stand-alone environment, such as on a single terminal.

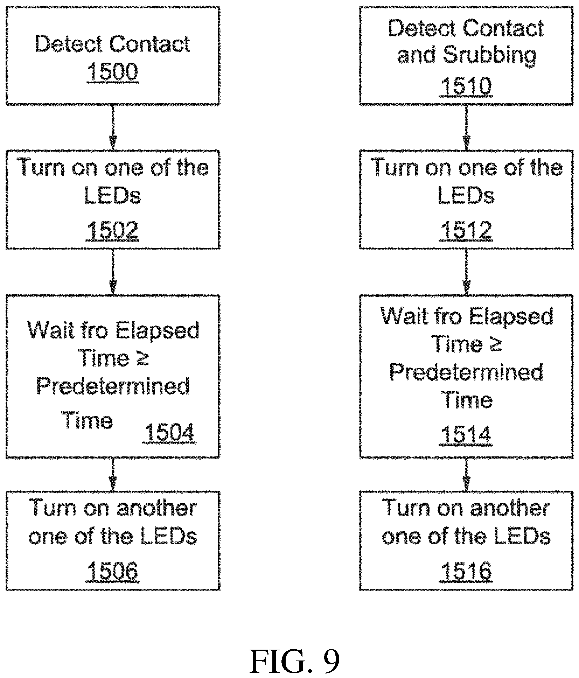

In some implementations, the integrated circuit 1000 may be coupled, such as by electrical connection to one or more LEDs. With reference to Figure 9, one of the LEDs may be turned on (1500) in response to contacts between the antiseptic applicator and the skin of the patient detected (1502) by the CSIC 1006. Next, another one of the LEDs may be turned on (1506) in response to the elapsed time being equal to or greater than the predetermined duration of time (1504). Alternatively, one of the LEDs may be turned on (1512) in response to both proper contacts detected by the CSIC and proper scrubbing motion detected (1510) the accelerometer. Next, another one of the LEDs may be turned on (1516) in response to the elapsed time being equal to or greater than the predetermined duration of time (1514). Other programming patterns for the one or more LEDs are possible.

Aspects of the present invention may be embodied in other specific forms without departing from its structures, methods, or other essential characteristics as broadly described herein and claimed hereinafter. The described embodiments and examples are to be considered in all respects only as illustrative, and not restrictive. The scope hereof is, therefore, indicated by the appended claims, rather than by the foregoing description.

## Claims

1.  A medical device (12) for sterilizing a body of a patient, comprising:

    an applicator body (38) that stores antiseptic solution;
    an antiseptic dispenser that dispenses the antiseptic solution;
    a capacitor (936) that abuts a surface of the antiseptic dispenser;
    measurement means for determining a capacitance between the capacitor and the body of the patient;
    **characterized in that** the medical device further comprises:

    a color-changing element (50) configured to indicate a duration of exposure to air; and
    a timer (24) configured to record an amount of time when the capacitance is approximately equal to or greater than a threshold capacitance.

2.  The medical device (12) of claim 1, further comprising a communication circuit (974) configured to transmit data collected from the measurement means.

3.  The medical device (12) of claim 2, wherein the communication circuit transmits the data using Bluetooth, Wi-Fi, Wi-Fi Direct, Near Field Communication, or Radio Frequency Identification.

4.  The medical device (12) of claim 1, further comprising a first indicator operatively coupled to the measurement means, wherein the first indicator is configured to provide indication when the capacitance is approximately equal to or greater than a threshold capacitance.

5.  The medical device (12) of claim 4, wherein the first indicator is a light emitting diode (18).

6.  The medical device (12) of claim 5, further comprising a second light emitting diode (18) operatively coupled to the measurement means, wherein the second light emitting diode (18) is configured to provide indication when the time exceeds a predetermined duration.

## Patentansprüche

1.  Medizinische Vorrichtung (12) zum Sterilisieren eines Patientenkörpers, umfassend: einen Applikatorkörper (38), der eine antiseptische Lösung enthält; einen antiseptischen Spender, der die antiseptische Lösung ausgibt; einen Kondensator (936), der an einer Oberfläche des antiseptischen Spenders anliegt; Messmittel zum Bestimmen einer Kapazität zwischen dem Kondensator und dem Körper des Patienten; **dadurch gekennzeichnet, dass** die medizinische Vorrichtung ferner umfasst: ein farbwechselndes Element (50), das so konfiguriert ist, dass es die Dauer der Einwirkung von Luft anzeigt; und einen Zeitgeber (24), der so konfiguriert ist, dass er eine Zeitspanne aufzeichnet, in der die Kapazität ungefähr gleich oder größer als eine Schwellenkapazität ist.

2. Medizinische Vorrichtung (12) nach Anspruch 1 ferner umfassend eine Kommunikationsschaltung (974), die so konfiguriert ist, dass sie von den Messmitteln erfasste Daten überträgt.

3. Medizinische Vorrichtung (12) nach Anspruch 2, wobei der Kommunikationsschaltkreis die Daten unter Verwendung von Bluetooth, Wi-Fi, Wi-Fi Direct, Nahfeldkommunikation oder Radiofrequenzidentifikation überträgt.

4. Medizinische Vorrichtung (12) nach Anspruch 1, die ferner einen ersten Indikator umfasst, der funktionell mit dem Messmittel gekoppelt ist, wobei der erste Indikator so konfiguriert ist, dass er anzeigt, wenn die Kapazität ungefähr gleich oder größer als eine Schwellenkapazität ist.

5. Medizinische Vorrichtung (12) nach Anspruch 4, wobei der erste Indikator eine Leuchtdiode (18) ist.

6. Medizinische Vorrichtung (12) nach Anspruch 5, die ferner eine zweite Leuchtdiode (18) umfasst, die funktionell mit dem Messmittel gekoppelt ist, wobei die zweite Leuchtdiode (18) so konfiguriert ist, dass sie eine Anzeige liefert, wenn die Zeit eine vorbestimmte Dauer überschreitet.

**Revendications**

1. Dispositif médical (12) pour la stérilisation du corps d'un patient, comprenant: un corps d'applicateur (38) qui stocke la solution antiseptique; un distributeur d'antiseptique qui distribue la solution antiseptique; un condensateur (936) qui s'appuie sur une surface du distributeur d'antiseptique; des moyens de mesure pour déterminer une capacité entre le condensateur et le corps du patient; **caractérisé en ce que** le dispositif médical comprend en outre: un élément changeant de couleur (50) configuré pour indiquer la durée de l'exposition à l'air; et une minuterie (24) configurée pour enregistrer une durée pendant laquelle la capacité est approximativement égale ou supérieure à une capacité seuil.

2. Dispositif médical (12) de la revendication 1 comprend en outre un circuit de communication (974) configuré pour transmettre les données recueillies par les moyens de mesure.

3. Dispositif médical (12) de la revendication 2, dans lequel le circuit de communication transmet les données à l'aide de Bluetooth, Wi-Fi, Wi-Fi Direct, Near Field Communication ou Radio Frequency Identification.

4. Dispositif médical (12) de la revendication 1 comprend en outre un premier indicateur couplé de manière opérationnelle aux moyens de mesure, dans lequel le premier indicateur est configuré pour fournir une indication lorsque la capacité est approximativement égale ou supérieure à une capacité seuil.

5. Dispositif médical (12) de la revendication 4, dans lequel le premier indicateur est une diode électroluminescente (18).

6. Dispositif médical (12) de la revendication 5, comprenant en outre une seconde diode électroluminescente (18) couplée de manière opérationnelle aux moyens de mesure, la seconde diode électroluminescente (18) étant configurée pour fournir une indication lorsque le temps dépasse une durée prédéterminée.

12 →

38

50

52

10

14

## FIG. 1

60 →

62

| Press an antiseptic applicator on skin of a patient |

| Remove the antiseptic applicator from the skin after a usage indicator indicates a period of time has elapsed |

64

## FIG. 2

FIG. 3

FIG. 4

FIG. 5

FIG. 6

Computer System 1300

FIG. 7

FIG. 8

Detect Contact
1500

Turn on one of the
LEDs
1502

Wait fro Elapsed
Time ≥
Predetermined
Time      1504

Turn on another
one of the LEDs
1506

Detect Contact
and Srubbing
1510

Turn on one of the
LEDs
1512

Wait fro Elapsed
Time ≥
Predetermined
Time
1514

Turn on another
one of the LEDs
1516

FIG. 9

**EP 3 758 786 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 15910318 B
- US 15377092 B
- WO 2017109519 A